Europäisches Patentamt

(19) European Patent Office

Office européen des brevets

(11) Publication number: 0 277 795
A2

(12) **EUROPEAN PATENT APPLICATION**

(21) Application number: 88300832.8

(22) Date of filing: 01.02.88

(51) Int. Cl.⁴: **C 08 F 8/32**
C 08 F 212/14, A 61 K 31/74

(30) Priority: 03.02.87 GB 8702318

(43) Date of publication of application:
10.08.88 Bulletin 88/32

(84) Designated Contracting States: DE FR GB IT

(71) Applicant: **Rohm and Haas Company**
**Independence Mall West**
**Philadelphia, Pennsylvania 19105 (US)**

(72) Inventor: **Sivieri, Franco**
**Via Degli Orti 18**
**I-24043 Caravaggio (BG) (IT)**

(74) Representative: **Tanner, James Percival et al**
**ROHM AND HAAS (UK) LTD. European Operations Patent**
**Department Lennig House 2 Mason's Avenue**
**Croydon CR9 3NB (GB)**

(54) **Process for preparation of cholestyramine.**

(57) Known processes for preparing cholestyramine involve chloromethylating and aminating unfunctionalized styrene/divinylbenzene beads in the presence of added halogenated hydrocarbon swelling solvent, typically ethylene dichloride. The use of such halogenated swelling solvent is disadvantageous because of toxicological implications.

The present invention provides a process for preparing cholestyramine by chloromethylating and aminating unfunctionalized styrene/divinylbenzene copolymer beads, the chloromethylation and amination being conducted in the absence of added halogenated hydrocarbon swelling sovent.

Furthermore, the cholestyramine produced by the process of the invention has improved palatability when compared with cholestyramine produced by known processes.

EP 0 277 795 A2

## Description

### PROCESS FOR PREPARATION OF CHOLESTYRAMINE

The present invention is concerned with a process for preparing cholestyramine free from halogenated organic solvent.

Cholestyramine is an anion exchange resin having pharmaceutical uses. As such pharmaceutical uses may involve oral adminstration of the cholestyramine, it is important for it to contain no toxic materials, e.g. impurities.

At present, cholestyramine is prepared by a process involving chloromethylation of unfunctionalized styrene/divinylbenzene copolymer beads using chloromethylating agents and eventually amination of the chloromethylated polymer, the chloromethylation and amination steps being conducted in the presence of added halogenated hydrocarbon swelling solvent, e.g. ethylene dichloride. The presence of swelling solvent has been considered to be necessary in order to achieve swelling of the unfunctionalized styrene/divinylbenzene copolymer beads and thereby allow their efficient chloromethylation. Halogenated hydrocarbon swelling solvents have been used to avoid reaction between the swelling solvent and the chloromethylating agents used in the chloromethylation.

Small amounts of the halogenated hydrocarbon swelling solvent have been observed, as an impurity, in the final cholestyramine product and this is, of course, disadvantageous because of toxicological implications.

There is, therefore, a need for a process which avoids the presence of such halogenated solvents in the final product. In view of this need, the obvious step was to try to remove the halogenated hydrocarbon solvent from the cholestyramine product. However, this proved to be both expensive and difficult to achieve.

We have now found that it is possible to prepare cholestyramine by a commercially attractive process in which the chloromethylation and amination steps are carried out in the absence of added halogenated hydrocarbon swelling solvent, and moreover that surprisingly leads to an improvement in the palatability of the cholestyramine product, a characteristic which has no apparent connection with the presence, in the cholestyramine, of halogenated hydrocarbon swelling solvent at the range of levels in current commercial products.

According to the present invention there is provided a process for preparing cholestyramine comprising chloromethylating unfunctionalized styrene/divinylbenzene copolymer beads using chloromethylether prepared in-situ, and aminating the resulting chloromethylated polymer, characterized in that the chloromethylation is conducted in the absence of added halogenated hydrocarbon solvent and the amination is conducted in the presence of non-halogenated solvent which is a solvent for the amine used in the amination, and which is capable of being absorbed into the chloromethylated copolymer beads.

One or more non-halogenated solvent may be present in the amination step. Suitable non-halogenated solvents for use in the amination step include esters such as ethyl acetate, propyl acetate and butyl acetate, ethers, alcohols, preferably alcohols having up to four carbon atoms such as methanol and ethanol, and water. Preferably the non-halogenated solvent present during the amination step is one or more ester, more preferably ethyl acetate.

The chloromethylether prepared in-situ in the chloromethylating step of the process is preferably prepared from a reaction mixture comprising formaldehyde, methanol and chlorosulfonic acid, preferably in the presence of excess water to aid in achieving the desired fluidity of the reaction mixture. For example, the molar ratio of water present in the chloromethylation step to the unfunctionalized styrene/divinylbenzene copolymer may be at least 1:1 and is preferably up to 4.5:1.

The chloromethylation of the unfunctionalized styrene/divinylbenzene resin is conveniently carried out in the presence of a suitable catalyst, for example, ferric chloride. The ferric chloride may be in the form of an aqueous solution, e.g. an approximately 40% by weight aqueous solution, the water aiding the fluidity of the chloromethylation reaction mixture.

In the embodiment of the invention in which the in-situ chloromethylation is conducted by preparing the chloromethylether from a reaction medium comprising formaldehyde, methanol and chlorosulfonic acid, and the thus formed chloromethylether is reacted with the styrene/divinylbenzene resin matrix in the presence of an aqueous ferric chloride solution, then one or more of the following parameters are preferably applied:-

(a) the molar ratio of ferric chloride to resin matrix is at least 0.001:1, e.g. at least 0.01:1, preferably at least 0.1:1, and is preferably up to 0.5:1;

(b) the molar ratio of formaldehyde to resin matrix is at least 2:1, e.g. at least 2.5:1, preferably at least 3:1, and is preferably up to 5:1, e.g. 3:1;

(c) the molar ratio of methanol to resin matrix is at least 2:1, e.g. at least 3:1, and is preferably up to 5:1, e.g. up to 3.5:1;

(d) the molar ratio of chlorosulfonic acid to resin matrix is at least 2:1, e.g. at least 3:1, and is preferably up to 5:1, e.g. up to 3:1.

The reaction product, obtained from the chloromethylation step, may be washed with water and aqueous sodium hydroxide in order to remove any excess chloromethylether therefrom, and/or subjected to steam stripping and/or organic solvent extraction to remove any organic material, such as styrene, adsorbed on the copolymer beads.

When ferric chloride is used as a catalyst during the chloromethylation, this can be removed by treatment with hydrochloric acid and heating, such treatment conveniently being conducted after the amination step. For example, hydrochloric acid having a concentration of approximately 32% may be added to the reaction product and the resultant mixture heated to approximately 90°C.

The aminating step of the process is conveniently carried out using a tertiary amine, preferably trimethylamine.

The non-halogenated solvent used in the amination step is preferably removable from the amination reaction product mixture by washing with water, by steam stripping or by chemical/biological hydrolysis/destruction.

The amination reaction product mixture may be subjected to one or both of:

(a) distillation, this distillation removing organic material(s) and, if present, water from the reaction product mixture; and

(b) steam stripping, e.g. for approximately 2 hrs, this steam stripping removing organic materials, e.g. the non-halogenated organic solvent, from the reaction product mixture.

The product obtained from the amination step may be washed with water, for example, at elevated temperature such as 95 to 97°C.

A preferred embodiment of the process according to the present invention comprises the following steps:-

(1). In-situ preparation of chloromethylether by reacting formaldehyde, methanol and chlorosulfonic acid in the presence of an aqueous solution of ferric chloride;

(2). Adding unfunctionalized styrene/divinylbenzene copolymer resin matrix to the reaction mixture from (1) above, thereby forming chloromethylated resin matrix;

(3). Washing the chloromethylated resin matrix obtained in (2) above to remove excess chloromethylether;

(4). Aminating the chloromethylated resin matrix using an amine, preferably using trimethylamine, in the presence of non-halogenated organic solvent which is a solvent for the amine and is capable of being absorbed into the chloromethylated copolymer beads; the solvent preferably being ethyl acetate;

(5). Removing non-halogenated organic solvent from the reaction product obtained in (4) above, for example, by distillation;

(6). Steam stripping the resin obtained in (5) above, e.g. for approximately two hours;

(7). Washing and acidifying with hydrochloric acid the resin obtained from (6) above, the hydrochloric acid removing the ferric chloride catalyst and related by-products, together with any unreacted amine.

It will, of course, be clear that steps (1) and (2) above may be conducted in the reverse order. That is, formaldehyde, methanol, chlorosulfonic acid and an aqueous solution of ferric chloride (i.e. the reactants for producing chloromethylether in-situ and the chloromethylation catalyst) may be added to an unfunctionalized styrene/divinylbenzene copolymer.

Water may be added during the distillation step (5) above in order to maintain the desired fluidity.

The process of the present invention is particularly suitable for the preparation of cholestyramine for pharmaceutical use since the process has the advantage that no added halogenated organic solvent is used and also has the unexpected advantage that the palatability of the resulting cholestyramine product is improved when compared with cholestyramine produced by known procedures using such halogenated organic swelling solvents. In this connection although we do not know the precise reason for the improvement in the palatability of the cholestyramine product obtained by the process of the present invention, it is known that halogenated solvents, when present at the normal levels (in fact not detectable) in commercially available cholestyramine, do not have any effect on the palatability of the final product. That is, the improved palatability of the cholestyramine product produced according to the present invention cannot be attributed to the absence, from the process, of added halogenated organic solvent.

The present invention will now be further illustrated by way of the following examples which are for illustrative purposes only and are not to be construed as imposing any limitation on the scope of the invention. In the examples all percentages are by weight.

Example 1

This is a comparative example describing the preparation of cholestyramine by a process involving the use of a chlorinated swelling solvent (ethylene dichloride or methylene dichloride) in the chloromethylation and amination steps.

A chloromethylating complex was initially prepared by dissolving 200g of chlorosulfonic acid in 120g of a formaldehyde (55%), methanol (35%) and water (10%) mixture and 150g of the chlorinated swelling solvent, the temperature being maintained at no greater than 40°C.

This chloromethylation complex was used to chloromethylate 100g of styrene-divinylbenzene copolymer beads, containing 0.5 to 2% by weight of the divinylbenzene crosslinking agent, by adding the chloromethylation complex to the copolymer beads and then gradually adding, to the resultant mixture, a 40% aqueous ferric chloride solution. The molar ratio of ferric chloride to the styrene-divinlybenzene copolymer was 0.01 to 0.5:1. The mixture was held at 18 to 30°C for a period of 6 to 14 hours and then washed with water and neutralized with caustic (i.e. aqueous sodium hydroxide).

The resultant solvent swollen chloromethylated copolymer were slurried with water and to this slurry was added 200g of a 40% aqueous solution of trimethylamine over a period of 3 hours while maintaining the temperature in the range of from 5 to 35°C.

The excess trimethylamine and chlorinated swelling solvent were then recovered by distillation.

3

The resultant copolymer beads were drained and washed with hot water to remove residual iron ions and trimethylamine.

About 700ml of a strong base anion exchange resin with an exchange capacity of 1.2 equivalents/liter or 4.0 equivalents/kilos were obtained. However, small amounts of the chlorinated swelling solvent were observed, as an impurity, in the resin product which is, as stated above, disadvantageous because of toxicological implications.

Example 2

This is a further comparative example and describes the preparation of cholestyramine without the presence of added halogenated swelling solvent during the chloromethylation/amination steps.

A chloromethylating complex was initially prepared by introducing, into a flask equiped with an agitator, thermometer and condenser, 230g of a formaldehyde (55%), methanol (35%) and water (10%) mixture. 74g of methanol and 64g of water were then added followed by the addition of 480g of chlorosulfonic acid, the chlorosulfonic acid being added slowly over a period of 4 to 6 hours and the temperature being from 37 to 41°C.

A chloromethylating complex was used to chloromethylate 100g of styrene-divinylbenzene copolymer beads, containing 0.5 to 2% by weight of the divinylbenzene crosslinking agent, under the same conditions as in Example 1.

After neutralization, the chloromethylated copolymer beads were slurried with deionized water and added to 74g of sodium chloride and 15g of 50% caustic and the resulting mixture was then mixed until the sodium chloride had completely dissolved.

To the resulting mixture was added, over a period of 2 hours, 200g of a 40% by weight aqueous solution of trimethylamine while maintaining the mixture at a temperature of from 0°C to 20°C. This mixture was then heated to recover residual trimethylamine and washed twice with hot water.

About 650ml of a strong base anion exchange resin were obtained with an exchange capacity of 1.25 equivalents/liter and 4.1 equivalents/kilo. However, the product was in the form of fragmented beads (0% whole beads) and major difficulties were experienced during the rinsing operation due to the poor resin integrity.

Example 3

This example describes the preparation of cholestyramine by a process in accordance with the present invention.

Chloromethylated styrene-divinylbenzene copolymer beads were prepared as in Example 2.

After washing of the chloromethylated copolymer beads with water and neutralization of the beads with caustic, the beads were slurried with 150g of ethyl acetate. The beads were allowed to swell for 1 hour and a 40% aqueous trimethylamine solution was then gradually added over a period of 2 hours, the temperature being maintained at from 0° to 20°C. This aminolysis reaction was completed by a distillation step to recover the organic solvent (i.e. ethyl acetate) and excess trimethylamine. The recovered organic solvent may be recycled and used during the aminolysis of a further batch of chloromethylated copolymer.

The aminolysed copolymer beads were then steam sparged for 2 hours to ensure an odour free product.

About 700g of anion exchange resin beads were obtained, the beads having good integrity and an exchange capacity of 1.32 equivalents/liter and 4.4 equivalents/kilo. Because of the good integrity, no difficulty was encountered in the rinsing operation.

Example 4

In this example several samples of cholestyramine, containing known concentrations of ethylene dichloride, were submitted to an experienced taste panel of three people who routinely check cholestyramine for palatability. The test panel were asked to rate the samples on a scale from 0 to 6, the higher the rating number, the less palatable the cholestyramine. The results obtained are given in the Table I.

## Table I

| | Reference Sample A | Sample A + 2.5 ppm EDC* | Sample A + 10 ppm EDC* | Sample A + 50 ppm EDC* |
|---|---|---|---|---|
| Average rating from Test Panel | 1 | 1 | 1 | 1 |

* = Ethylene dichloride

From the above Table it can be seen that the palatability of cholestyramine is not affected by varying levels of halogenated solvent, in this case ethylene dichloride. Consequently, the improved palatability of the cholestyramine product produced according to the present invention cannot be attributable to the absence, from the process, of added halogenated organic swelling solvent.

Cholestyramine produced by known processes typically has a rating of 1 to 2 on the above rating scale, whereas cholestyramine produced in accordance with the present invention has a rating of 0 to 1 on the same scale. This represents a significant improvement in palatability for the cholestyramine produced according to the invention, when compared with commercially available cholestyramine.

## Claims

1. A process for preparing cholestyramine comprising chloromethylating unfunctionalized styrene/divinylbenzene copolymer beads using chloromethylether prepared in-situ, and aminating the resulting chloromethylated copolymer using amine, characterized in that the chloromethylation is conducted in the absence of added halogenated hydrocarbon solvent and the amination is conducted in the presence of non-halogenated solvent which is a solvent for the amine used in the amination, and which is capable of being absorbed into the chloromethylated copolymer beads.

2. A process as claimed in Claim 1, in which the chloromethylether is prepared in-situ from a reaction mixture comprising formaldehyde, methanol and chlorosulfonic acid, the preparation of the chloromethylether preferably being conducted in the presence of excess water.

3. A process as claimed in Claim 2, in which the in-situ preparation of the chloromethylether is conducted in the presence of water, the molar ratio of water to the unfunctionalized styrene/divinylbenzene copolymer being at least 1:1, and preferably being up to 4.5:1.

4. A process as claimed in any of Claims 1 to 3, in which the chloromethylation of the unfunctionalized styrene/divinylbenzene copolymer is carried out in the presence of a catalyst, preferably ferric chloride.

5. A process as claimed in any of Claims 1 to 4, in which the reaction product obtained from the chloromethylation step is washed with water and aqueous sodium hydroxide to remove any excess chloromethyl ether therefrom, and/or is subjected to steam stripping and/or organic solvent extraction to remove any organic material adsorbed on the copolymer beads.

6. A process as claimed in Claim 1, in which the in-situ chloromethylation is conducted by preparing the chloromethylether from a reaction mixture comprising formaldehyde, methanol and chlorosulfonic acid, the thus formed chloromethylether is reacted with the styrene/divinylbenzene copolymer in the presence of an aqueous ferric chloride solution, and one or more of the following parameters is applied:-
    (a) the molar ratio of ferric chloride to copolymer is at least 0.001:1, and preferably is up to 0.5:1;
    (b) the molar ratio of formaldehyde to copolymer is at least 2:1, and preferably is up to 5:1;
    (c) the molar ratio of methanol to copolymer is at least 2:1, and preferably is up to 5:1; and
    (d) the molar ratio of chlorosulfonic acid to copolymer is at least 2:1, and preferably is up to 5:1.

7. A process as claimed in any of Claims 1 to 6, in which the non-halogenated solvent comprises one or more of esters, preferably ethyl acetate, propyl acetate or butyl acetate, ethers, alcohols, preferably alcohols having up to 4 carbon atoms, and water.

8. A process as claimed in any of Claims 1 to 7, in which the amine used in the aminating step is a tertiary amine, preferably trimethylamine.

9. A process as claimed in any of Claims 1 to 8, in which the amination reaction product mixture is subjected to one or both of:-

a) distillation to remove organic material(s) and, if present, water from the reaction product mixture; and

b) steam stripping, preferably for approximately two hours, to remove organic material(s) from the reaction product mixture.

10. A process as claimed in Claim 1, which comprises:-

1) either

(i) in-situ preparation of chloromethylether by reacting formaldehyde, methanol and chlorosulfonic acid in the presence of an aqueous solution of ferric chloride, and then adding the unfunctionalized styrene/divinylbenzene copolymer to the resultant reaction mixture, thereby forming chloromethylated copolymer, or

(ii) adding formaldehyde, methanol, chlorosulfonic acid and an aqueous solution of ferric chloride to the unfunctionalized styrene/divinylbenzene copolymer; thereby forming chloromethylether in-situ which reacts with the unfunctionalized copolymer to form chloromethylated copolymer;

2) washing the chloromethylated copolymer obtained in (1) above to remove excess chloromethylether;

3) aminating the chloromethylated copolymer using an amine, preferably trimethylamine, in the presence of non-halogenated organic solvent which is a solvent for the amine and is capable of being absorbed into the chloromethylated copolymer beads; the solvent preferably being ethyl acetate;

4) removing non-halogenated organic solvent from the reaction product obtained in (3) above, for example, by distillation;

5) steam stripping the resin obtained in (4) above, for example, for approximately two hours; and

6) washing and acidifying with hydrochloric acid the resin obtained from (5) above, the hydrochloric acid removing the ferric chloride and related by-products together with any unreacted amine.